# EUROPEAN PATENT APPLICATION

(11) **EP 4 851 271 A1**
(43) Date of publication of application: **09.12.2026**
(21) Application number: 25749043.3
(22) Date of filing: 31.01.2025
(51) Int. Cl.: C12M 1/12, C12M 1/00, C12M 3/00, C12M 1/32

(54) **PREFABRICATED MULTI-WELL PLATE FOR CULTURING SINGLE MICROORGANISM IN LIQUID MEDIUM**

(30) Priority: 30.01.2024 KR 20240014047; 24.01.2025 KR 20250011311
(71) Applicant: Bioneer Corporation, Daejeon 34013 (KR)
(72) Inventor: PARK, Han-Oh, Sejong 30151 (KR)
(74) Representative: Primrose Oy
(86) International application number: PCT/KR2025/001560
(87) International publication number: WO 2025/165140

(57) **Abstract**

The purpose of the present disclosure is to provide a multi-well plate for culturing a single microorganism, capable of conveniently and rapidly dividing and culturing a culture medium containing a single microorganism in a plurality of multi-wells. The multi-well plate of the present disclosure is economical because a special device is not additionally used, and a method for culturing a single microorganism using same can efficiently culture a single microorganism in multi-wells without a process of diluting a culture medium in each well.

## Description

### TECHNICAL FIELD

The present invention relates to a prefabricated multi-well plate for culturing a single microorganism in a liquid medium and a method for culturing a single microorganism using the same.

### BACKGROUND ART

The continued emergence of antibiotic-resistant bacteria is the biggest threat to global health, and it is warned that by 2050, 10 million people will die due to antibiotic-resistant bacteria infection every year. Therefore, it is a very important task at hand to develop a new antibiotic. Most antibiotics developed so far have been developed from those produced by microorganisms. However, there were limitations in discovering new microorganisms to make antibiotics. Traditionally, single isolation of microorganisms has been performed using an agarose solid medium, and the method has a problem in screening new microorganism species since many microorganisms which are cultured only in a liquid phase were not able to be singly isolated.

Meanwhile, microalgae of which the number of species known to date is expected to be 200,000 to 800,000, and the like are the precious resources of the earth which produces 15,000 natural substances including various antibiotics and physiologically active substances. In order to use the microalgae, single isolation should be performed, but most of the single isolated microalgae does not grow in a solid phase culture. Since a single isolation method using a current single isolation method using capillary tubes, and the like is labor-intensive, there are only thousands of single microalgae held by the microalgae bank.

In the conventional isolation identification of microorganisms, a method of obtaining a single colony by performing smear and culture using glass rods, glass beads, and the like in a solid medium such as agarose in a petri dish has been generally used. However, in a conventional culture method using a solid medium, it was reported that only less than 1% of known microorganisms was able to be cultured. Meanwhile, a culture method using a liquid medium is a method of producing a droplet using a solution in which microorganisms are dispersed, and purely isolating and identifying microorganisms in this process, and since it is difficult to separately classify droplets in which microorganisms are captured, improvement is required. Besides, a method of liquid culturing a single type of microorganism by performing dilution continuously with a culture medium using a multi-well plate such as a 96-well plate may be used, but much effort is needed when manually performed.

Accordingly, the development of an efficient culture plate for efficient single isolation of various microorganisms is required.

### DISCLOSURE

### Technical Problem

An object of the present disclosure is to provide a multi-well plate for culturing a single microorganism, which may independently culture a single microorganism in a liquid phase by forming a plurality of isolated multi-wells.

Another object of the present disclosure is to provide a method for culturing a single microorganism, which may culture a single microorganism in a liquid phase without a process of diluting a culture medium in each well of the multi-well plate and does not allow the growing microorganisms in each well to be cross-contaminated with each other.

### Technical solution

In one general aspect, a multi-well plate for culturing a single microorganism includes: a lower container including a bottom, a plurality of side walls, and a plurality of first connection parts placed on the side walls; a perforated plate including a plurality of through holes and a plurality of second connection parts which are formed in an edge and independently connected to the first connection parts; and a lid detachably coupled to a top of the perforated plate.

The lower container may include one or more partition walls which divide across the container and one or more housing grooves placed on the partition wall, and the perforated plate may include one or more divided regions which divide the through holes across the plate and one or more protruding parts which are placed in the lower portion of the divided region and combine with the housing groove.

The lower container may further include an elastic body formed on the surfaces of the bottom, the side wall, and the partition wall.

The elastic body may include polypropylene.

The elastic body may have a hardness of 10 to 40.

The elastic body may have a thickness of 1.0 to 2.5 mm.

The height (a) of the through hole may satisfy the following Relation 1: [Relation 1] ${h}_{o} - {h}_{e} < a < {h}_{o}$ wherein
a is a height of the through hole, hₒ is a height of the lower container, and hₑ is a thickness of the elastic body.

The through hole may have a diameter of 1.0 to 3.0 mm.

The lid may include a gas permeable breathable membrane.

The cross-sectional shape of the through hole may be a quadrangular, hexagonal, or circular shape.

In another general aspect, a method for culturing a single microorganism includes: injecting a culture medium including a single microorganism into a lower container including a bottom, a plurality of side walls, and first connection parts placed on the side walls; connecting a perforated plate including a plurality of through holes and a plurality of second connection parts which are formed in an edge and independently connected to the first connection parts to the lower container to seal the culture medium in each of the through holes; and covering the top of the perforated plate with a lid and performing culturing.

The lower container may include one or more partition walls which divide across the container and one or more housing grooves placed on the partition wall, and the perforated plate may include one or more divided regions which divide the through holes across the plate and one or more protruding parts which are placed in the lower portion of the divided region and combine with the housing groove.

The lower container may further include an elastic body formed on the surfaces of the bottom, the side wall, and the partition wall.

The number (N) of single microorganisms injected into the lower container may satisfy the following Relation 2: [Relation 2] $N \geq 0.1 a \left(unit:CFU/\left(a*V\right)\right) \left(100<a<50000\right)$ wherein a is the number of wells, and V is an individual capacity of the well.

### Advantageous Effects

The multi-well plate for culturing a single microorganism of the present disclosure may conveniently and rapidly divide a culture medium including a single microorganism into a plurality of multi-wells and perform culturing, is economical since a special device is not additionally used, and may efficiently culture the single microorganism in the multi-well without a process of diluting the culture medium in each well.

The method for culturing a single microorganism of the present disclosure may independently culture the single microorganism in each well, since the culture medium is divided into a plurality of wells in a single step and completely separated.

### DESCRIPTION OF DRAWINGS

FIG. 1 is a perspective view of a multi-well plate according to an implementation example of the present disclosure.
FIG. 2 is a perspective view of a multi-well plate according to another implementation example of the present disclosure.
FIG. 3 shows the front and back of a perforated plate of the multi-well plate of the present disclosure.
FIG. 4 shows the front of a lower container of the multi-well plate of the present disclosure.
FIG. 5 shows a process of connecting the lower container and the perforated plate of the multi-well plate of the present disclosure.
FIG. 6 shows an appearance of an elastic body (green) when the lower container and the perforated plate of the multi-well plate of the present disclosure are connected, in which a is a height of a through hole, hₒ is a height of the lower container, and hₑ is a thickness of the elastic body.
FIG. 7 shows housing grooves placed on a partition wall in the lower container of the multi-well plate of the present disclosure.

### BEST MODE

Hereinafter, exemplary embodiments of the present invention will be described in detail with reference to the accompanying drawings so as to be easily practiced by a person skilled in the art to which the present invention pertains. The present invention may be implemented in various different forms and is not limited to the exemplary embodiments described herein.

Conventionally, a single species of microorganisms was cultured by continuously diluting a culture medium using a multi-well plate in order to culture a single microorganism in a liquid medium, but much effort is needed when manually performed, and it is less efficient. Accordingly, the present disclosure provides a multi-well plate for culturing a single microorganism, which may culture a single microorganism in a liquid phase without a process of diluting a culture medium in each well and does not allow the growing microorganisms in each well to be cross-contaminated with each other.

The multi-well plate for culturing a single microorganism of the present disclosure may include a lower container 100 including a bottom 110, a plurality of side walls 120, and a plurality of first connection parts 130 placed on the side walls; a perforated plate 200 including a plurality of through holes 210 and a plurality of second connection parts 230 which are formed in an edge and independently connected to the first connection parts; and a lid 300 detachable from the top of the perforated plate.

The multi-well plate for culturing a single microorganism may form a multi-well for culturing microorganisms, since the lower container 100 and the perforated plate 200 are adhered due to the connection of the first connection part 130 and the second connection part 230 so that each through hole 210 is strongly adhered to the bottom 110 of the lower container 100, and thus, the single microorganisms may be independently cultured without cross-contamination with each other in each well.

Specifically, the lower container 100 may include 4 side walls 120 and 1 to 3 first connection parts 130 in each side wall, and the number of the second connection parts 230 of the perforated plate 200 may be the same as the number of the first connection parts 130.

In addition, the bottom 110 of the lower container 100 may have a thickness of 1.0 to 4.0 mm, specifically 1.0 to 3.0 mm, and more specifically 1.5 to 2.5 mm. Herein, when the thickness of the bottom 110 is smaller than the range described above, there is a risk of deformation when fastening the lower container 100 and the perforated plate 200.

Specifically, the perforated plate 200 may have 100 to 50000 through holes 210, but is not limited as long as many microorganisms may be isolated. In addition, the through hole 210 may have a diameter of 0.5 to 5.0 mm, more specifically 0.5 to 4.0 mm, or 1.0 to 3.0 mm. The size of the through hole 210 is determined considering recovery of microorganisms from the well where the microorganisms were grown, and when the diameter of the through hole 210 is smaller than the range described above, it is not easy to recover the microorganisms cultured inside the well, and when the diameter is larger than the range described above, it is difficult to perform pure culturing of many species of microorganisms at once.

According to an implementation example, the cross-sectional shape of the through hole 210 may be a quadrangular, hexagonal, or circular shape, but is not limited thereto.

Fine holes may be formed on the top of the through hole 210, and the fine holes may decrease leakage of gas occurring inside the through hole 210 and inflow of gas from the outside.

The lid 300 performs a contamination prevention function since it is provided in a state of covering the top of the perforated plate 200 and being sterilized. In addition, it performs functions of preventing contamination from external environments such as users' hands, gas, pollutants and preventing evaporation of liquid occurring during culturing.

According to an implementation example, the lid 300 may be formed of a transparent material. Due to the transparent material, it may be checked whether cells are growing inside the multi-well plate by turbidity using light, and a cell proliferation rate may be checked depending on the turbidity of the solution.

According to another implementation example, the lid 300 may be formed of a gas permeable breathable membrane. The breathable membrane may perform a gas exchange function into the well and help microorganism culture. In addition, when microorganisms cultured inside the well are taken out, a microorganism culture medium may be taken out by penetrating the breathable membrane with a syringe needle. The breathable membrane may be fixed to the upper surface of the perforated plate 200 and used.

Meanwhile, in order to spread and inject the culture medium well in/into the lower container 100 and the plurality of through holes 210, the inner surface of the lower container, the surface of the perforated plate 200, and the through hole 210 may be hydrophilically surface-treated by a plasma or chemical treatment, and thus, the microorganisms and the culture medium may be naturally and uniformly injected from the lower container into the through hole 210.

According to an implementation example, the lower container 100 includes one or more partition walls 140 which divide across the container and one or more housing grooves 150 placed on the partition wall 140, and the perforated plate 200 may include one or more divided regions 240 which divide the through holes 210 across the plate and one or more protruding parts 250 which are placed in the lower portion of the divided region 240 and connected to the housing groove 150.

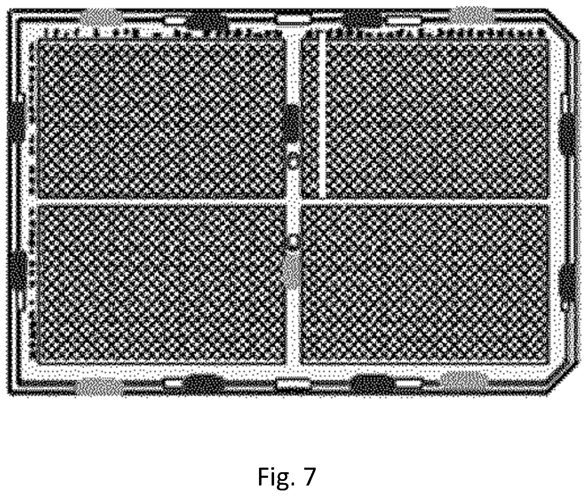

n (0<n<10, n=1,2,3,4,5,⊚) housing grooves 150 placed on the partition wall 140 may be formed at 1/(n+1), 2/(n+1), 3/(n+1), ..., n/(n+1) points of the length of the partition wall 140, respectively, and the protruding part 250 may be formed in the lower portion of the divided region 240 of the perforated plate 200 corresponding to the positions of the plurality of housing grooves 150. Specifically, as shown in FIG. 7, the red and blue housing grooves 150 placed on the partition wall 140 may be formed at 1/2 position of the length of the partition wall marked in yellow. The perforated plate 200 is adhered to the lower container 100 more stably by the connection of the housing groove 150 and the protruding part 250, and the movement of substances between wells of the housing groove 150 may be blocked.

Specifically, the lower container 100 may include 1 or 2 partition walls 140 and 1 to 5 housing grooves 150 placed on the partition wall 140, and specifically, the lower container 100 includes 2 partition walls 140 and 2 housing grooves 150 placed in one of the partition walls 140. Herein, the perforated plate 200 may include 1 or 2 divided regions 240, and the protruding part 250 is an area corresponding to the housing groove 150 placed in the perforated plate 140 of the lower container 100 and may be placed in the lower portion of the divided region 240.

In addition, the material forming the multi-well plate is not particularly limited and may use a material commonly used in the microorganism culture. For example, a transparent material such as a polystyrene resin, a polyester resin, a polyethylene resin, a polyethylene terephthalate resin, a polypropylene resin, an acryl resin, a polycarbonate resin, an epoxy resin, and a vinyl chloride resin may be used, resin materials including at least one of the resins which is hydrophilically surface-treated or transparent inorganic materials such as glass or quartz may be used, and it may be materials allowing the well where microorganisms grow to be identified.

According to an implementation example, the lower container 100 may further include an elastic body 160 formed on the surfaces of the bottom 110, the side wall 120, and the partition wall 140. Specifically, the elastic body 160 may be formed on one surface where the bottom 110, the side wall 120, and the partition wall 140 of the lower container 100 are in contact with each other, and thus, when each through hole 210 is in contact with the bottom 110 of the lower container 100 in the connection between the perforated plate 200 and the lower container 100, a plurality of wells are formed by a locking action by the elastic body 160. Liquid movement is blocked between the formed wells, thereby preventing contamination of microorganisms cultured inside the well.

According to an implementation example, the elastic body may include a polypropylene copolymer. The polypropylene copolymer may be a copolymer with an α-olefin monomer, and the olefin monomer may include at least one selected from the group consisting of ethylene, 1-butene, 1-pentene, 1-hexene, and a combination thereof.

The polypropylene copolymer may include homo- and copolymer. Specifically, the polypropylene copolymer may include at least one selected from the group consisting of block polypropylene, random polypropylene, homopolypropylene, highly crystalline block polypropylene, highly crystalline homopolypropylene, metocene polypropylene, metallocene polypropylene, and a combination thereof.

The polypropylene copolymer may have a weight average molecular weight (Mw) of 200,000 to 500,000 g/mol, and specifically a lower limit of 250,000 or 300,000 g/mol and an upper limit of 500,000, 450,000, or 400,000 g/mol.

Specifically, the polypropylene copolymer may have an isotactic index (I.I) of 97.5 to 99.5%. In addition, the polypropylene copolymer may have a density of 0.80 to 0.95g/cm³, specifically 0.85 to 0.92 g/cm³, and more specifically 0.89 to 0.92 g/cm³.

The polypropylene copolymer may have a melt flow index measured at a load of 2.16 kg at 230°C of 0.1 g/10 min to 1.0 g/10 min, and specifically 0.5 g/10 min to 1.0 g/10 min**.**

According to an implementation example, the elastic body may have a hardness (A) of 10 to 40, specifically 10 to 30 or 10 to 25.

According to an implementation example, the elastic body may have a thickness of 1.0 to 2.5 mm, specifically 1.5 to 2.5 mm. Herein, when the thickness is smaller than the range described above, the locking action by the elastic body may be weak, and flatness may be lowered when the thickness is large.

According to an implementation example, the height (a) of the through hole 210 of the perforated plate may satisfy the following Relation 1: [Relation 1] ${h}_{o} {- h}_{e} {< a < h}_{o}$ wherein
a is the height of the through hole 210, hₒ is the height of the lower container 100, and hₑ is the thickness of the elastic body 160.

Specifically, when the height (hₒ-hₑ) of an inner housing space of the lower container 100 is lower than the height (a) of the through hole 210, the perforated plate 200 is mounted in the lower container 100, pressure may be applied to the elastic body 160, and thus, the thickness of the elastic body 160 on the bottom 110 of the lower container 100 is decreased so that the through hole 210 may be completely adhered to the bottom 110 of the lower container 100.

In addition, the present disclosure provides a method for culturing a single microorganism, which may culture a single microorganism in a liquid phase without a process of diluting a culture medium in each well of the multi-well plate and does not allow the microorganisms growing in each well to be cross-contaminated with each other.

Specifically, the method for culturing a single microorganism may include: injecting a culture medium including a single microorganism into a lower container 100 including a bottom 110, a plurality of side walls 120, and a plurality of first connection parts 130 placed on the side walls 120; connecting a perforated plate 200 including a plurality of through holes 210 and a plurality of second connection parts 230 which are formed in an edge 220 and independently connected to the first connection parts 130 to the lower container 100 to seal the culture medium in each of the through holes 210; and covering a lid 300 from the top of the perforated plate 200 and performing culturing.

The method for culturing a single microorganism of the present disclosure may improve user convenience, by rapidly and simply injecting microorganisms and a culture medium into the lower container 100 of the multi-well plate.

According to an implementation example, the lower container 100 includes one or more partition walls 140 which divide across the container and one or more housing grooves 150 placed on the partition wall 140, and the perforated plate may include one or more divided regions 240 which divide the through holes 210 across the plate and one or more protruding parts 250 which are placed in the lower portion of the divided region 240 and connected to the housing groove 150.

According to an implementation example, the lower container 100 may further include an elastic body 160 formed on the surfaces of the bottom 110, the side wall 120, and the partition wall 140.

Specifically, the perforated plate 200 including the plurality of through holes 210 is separated from the lower container 100 and hung on an engaging protrusion of the connection part, before use, and when the microorganisms and the culture medium are first injected into the lower container 100 in a horizontal workbench during use and then the perforated plate 200 is adhered to the lower container 100, the elastic body 160 between the lower container 100 and the perforated plate 200 is pressed and adhered, and then pressed by the tight-fitting engaging protrusion of a connecting means to completely separate each through hole 210, thereby forming the multi-well.

According to an implementation example, the number (N) of single microorganisms injected into the lower container 100 may satisfy the following Relation 2: [Relation 2] $N \geq 0.1 a \left(unit:CFU/\left(a*V\right)\right) \left(100<a<50000\right)$ wherein a is the number of wells, and V is an individual capacity of the well.

Specifically, in Relation 2, the lower limit of N may be 0.1a, 0.2a, 0.3a, 0.4a, 0.5a, 0.6a, 0.7a, 0.8a, 0.9a, or 1.0a, and the upper limit may be 1.5a, 1.4a, 1.3a, 1.2a, 1.1a, or 1.0a.

When the microorganisms satisfying the concentration are injected into the lower container 100, 1 CFU or more of microorganisms may be injected into each well after mounting the perforated plate 200.

Herein, when the number of microorganisms is more than the number of multi-wells, other microorganisms grow simultaneously in many wells, and thus, the number of microorganisms may be less than the number of well, and specifically, when the number of bacteria included is 10% of the number of wells, it is effective for culturing a single microorganism.

As such, the method for culturing a single microorganism of the present disclosure may form several independent environments in which microorganisms may be cultured only by connection between the perforated plate 200 and the lower container 100 at once, and thus, allows convenient microorganism culture.

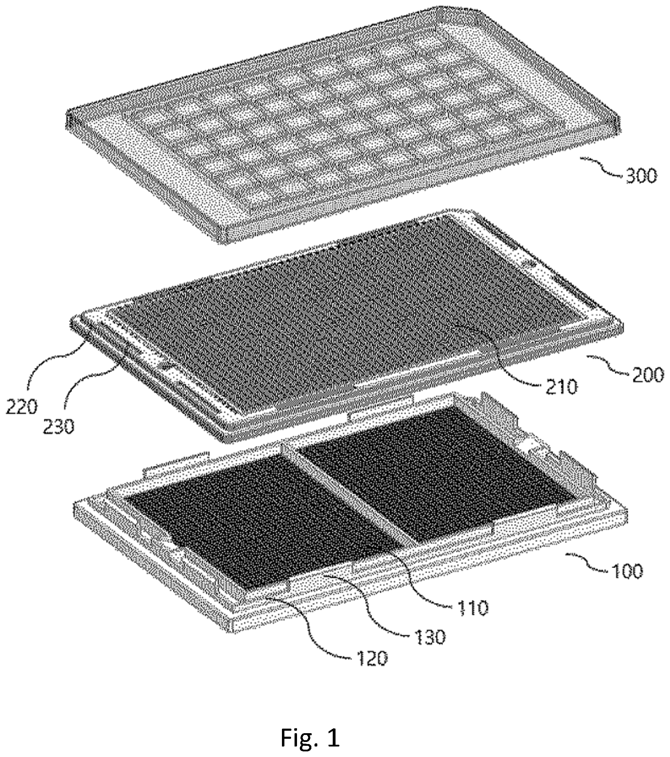

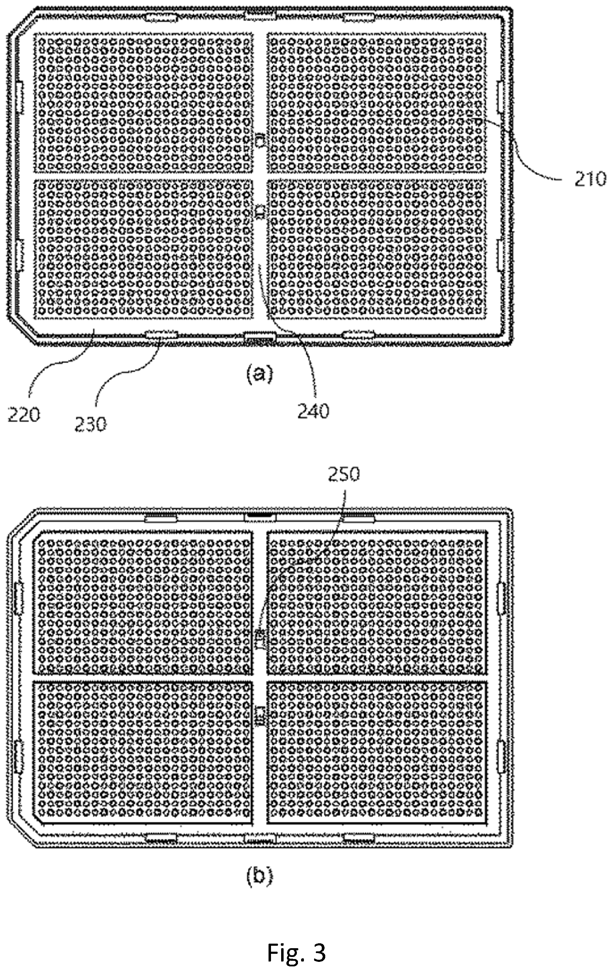

Hereinafter, the present disclosure will be described in detail referring to FIGS. 1 to 7.

Referring to FIG. 7, the multi-well plate for culturing a single microorganism according to an implementation example of the present disclosure is an integrated body of the lower container 100, the perforated plate 200, and the lid 300 which are sequentially assembled and may be used for culturing a single microorganism. Specifically, the lower container 100 may include a bottom 110, a plurality of side walls 120, and a plurality of first connection parts 130 placed on the side walls 120, and the perforated plate 200 may include a plurality of through holes 210 and a plurality of second connection parts 230 formed in an edge 220.

When the perforated plate 200 is connected from the top of the lower container 100, a plurality of first connection parts 130 placed on the side walls 120 of the lower container 100 and a plurality of second connection parts 230 formed in the edge 220 of the perforated plate 200 are independently connected to each other, so that the perforated plate 200 may be mounted on the lower container 100.

Specifically, the first connection part 130 having a protruding shape and the second connection part 230 having a housing groove shape are connected to each other, so that the perforated plate 200 is mounted on the lower container 100 and connected.

The perforated plate 200 is formed of a plurality of through holes 210. The cross-sectional shape of the through hole 210 may be any shape which may be separated from the surrounding and culture microorganisms. That is, it is shown in the drawing that the well has a uniform quadrangle shape, but is not limited thereto, and may be, for example, a pentagonal, hexagonal, circular shapes, and the like, and the shape and size may be changed considering the size and type of cultured microorganisms.

The microorganisms and the culture medium may be injected into the lower container 100 through an inlet positioned on one side or near the corner. Alternatively, the microorganisms and the culture medium may be directly injected into the center inside the lower container. Herein, the users may use liquid handling equipment such as a pipette and syringe for injecting the microorganisms and the culture medium into the inlet.

The inlet may be installed slantingly, and the slanting inlet performs a function of easily injecting or discharging the culture medium. In addition, one or more inlets may be provided near the one surface or edge of the lower container.

The lower container 100 may include a rib formed at a certain height on the inner surface of the side wall. More specifically, the rib may be formed along the inner transverse axis of the lower container. The user may apply a physical force such as shaking the lower container from side to side so that the microorganisms may uniformly spread inside the lower container, after injecting the culture medium and the microorganisms, and herein, overflowing of the culture medium to the outside may be prevented by the rib installed at a certain height inside the lower container. In addition, the injection amount of the culture medium may be checked by marking a rib or dotted line at a certain height inside the side of the lower container.

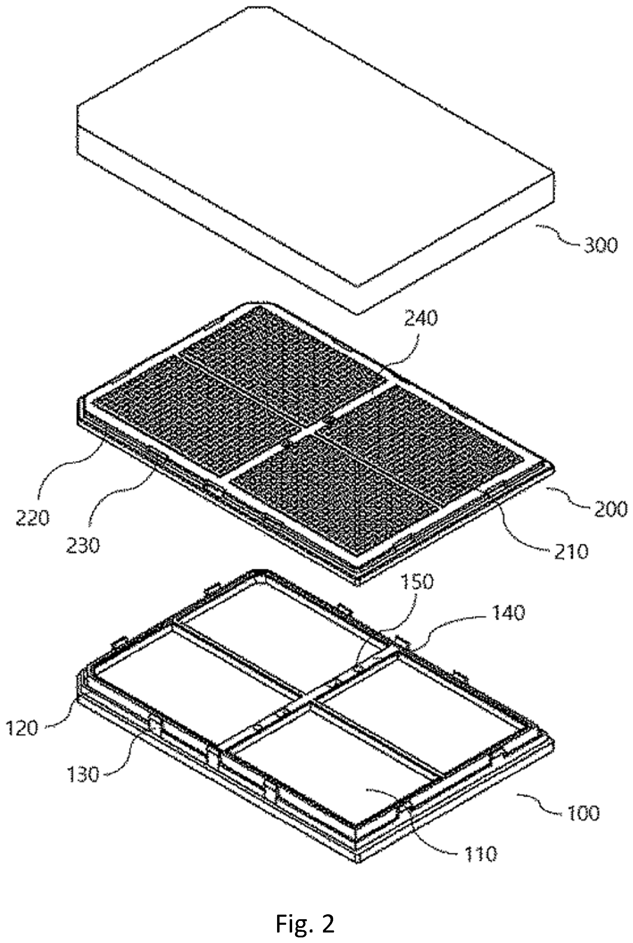

Referring to FIGS. 2 to 4, the multi-well plate for culturing a single microorganism according to another implementation example of the present disclosure includes one or more partition walls 140 which divide across the container and one or more housing grooves 150 placed on the partition wall 140, and the perforated plate may include one or more divided regions 240 which divide the through holes 210 across the plate and one or more protruding parts 250 which are placed in the lower portion of the divided region 240 and connected to the housing groove 150.

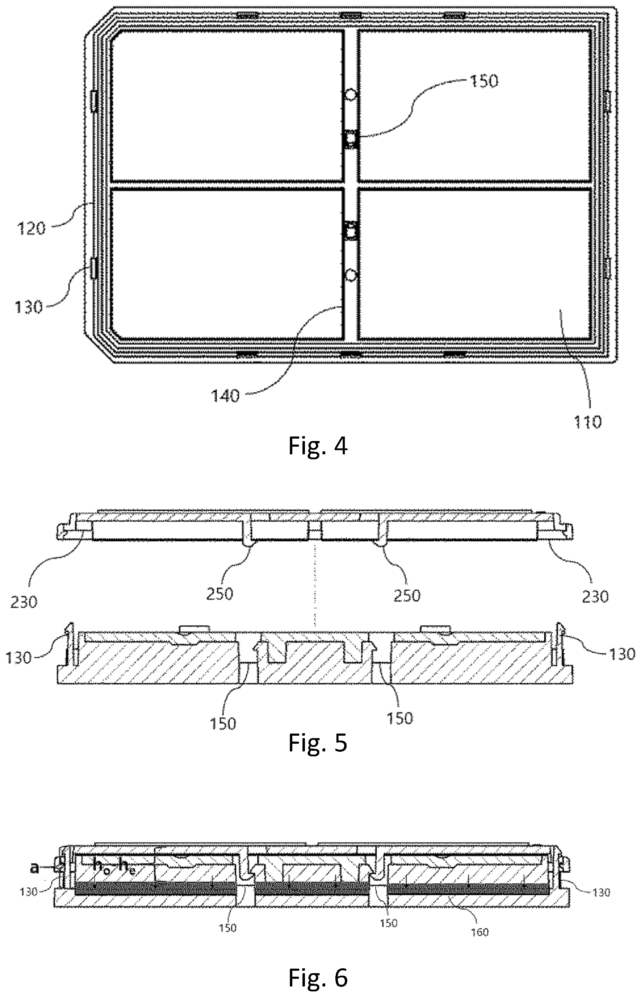

Referring to FIG. 5, when the perforated plate 200 is mounted on the lower container 100, the protruding part 250 placed on the lower portion of the divided region 240 of the perforated plate 200 may be housed and connected to the housing groove 150 of the partition wall 140 of the lower container 100. Accordingly, the perforated plate 200 may be more stably adhered and connected to the lower container 100, and substance movement between wells of the housing groove 150 may be blocked.

In addition, the lower container 100 may be formed by the elastic body 160 adhered and attached to the surfaces of the bottom 110, the side wall 120, and the partition wall 140. Specifically, when the perforated plate 200 is mounted on the lower container 100, the through hole 210 of the perforated plate 200 is adhered to the elastic body 160 formed on the bottom of the lower container 100, so that a space between wells of the through hole 210 may be completely sealed, and movement of a culture medium between wells may be blocked.

Referring to FIG. 6, the height (a) of the through hole 210 of the perforated plate 200 may satisfy the following Relation 1: [Relation 1] ${h}_{o} - {h}_{e} < a < {h}_{o}$ wherein
a is a height of the through hole, hₒ is a height of the lower container, and hₑ is a thickness of the elastic body.

When the height (hₒ-hₑ) of the inner housing space is lower than the height (a) of the through hole, the perforated plate 200 is mounted in the lower container 100, pressure may be applied to the elastic body 160, and thus, the thickness of the elastic body 160 on the bottom 110 of the lower container is decreased so that the through hole 210 may be completely adhered to the bottom 110 of the lower container.

Hereinabove, although the present invention has been described by specified matters and specific exemplary embodiments, they have been provided only for assisting in the entire understanding of the present invention, and the present invention is not limited to the exemplary embodiments. Various modifications and changes may be made by those skilled in the art to which the present invention pertains from the description.

Therefore, the spirit of the present invention should not be limited to the above-described exemplary embodiments, and the following claims as well as all modifications equal or equivalent to the claims are intended to fall within the scope and spirit of the invention.
100: Lower container
110: Bottom
120: Side wall
130: First connection part
140: Partition wall
150: Housing groove
160: Elastic body
200: Perforated plate
210: Through hole
220: Edge
230: Second connection part
240: Divided region
250: Protruding part
300: Lid

## Claims

1. A multi-well plate for culturing a single microorganism comprising:
a lower container including a bottom, a plurality of side walls, and a plurality of first connection parts placed on the side walls;
a perforated plate including a plurality of through holes and a plurality of second connection parts which are formed in an edge and independently connected to the first connection parts; and
a lid detachably coupled to a top of the perforated plate.

2. The multi-well plate for culturing a single microorganism of claim 1,
wherein the lower container includes one or more partition walls which divide across the container and one or more housing grooves placed on the partition wall, and
the perforated plate includes one or more divided regions which divide the through holes across the plate and one or more protruding parts which are placed in a lower portion of the divided region and connected to the housing groove.

3. The multi-well plate for culturing a single microorganism of claim 2, wherein the lower container further includes an elastic body formed on surfaces of the bottom, the side wall, and the partition wall.

4. The multi-well plate for culturing a single microorganism of claim 3, wherein the elastic body includes polypropylene.

5. The multi-well plate for culturing a single microorganism of claim 3, wherein the elastic body has a hardness of 10 to 40.

6. The multi-well plate for culturing a single microorganism of claim 3, wherein the elastic body has a thickness of 1.0 to 2.5 mm.

7. The multi-well plate for culturing a single microorganism of claim 1, wherein a height (a) of the through hole satisfies the following Relation 1: [Relation 1] ${h}_{o} - {h}_{e} < a < {h}_{o}$ wherein
a is a height of the through hole, hₒ is a height of the lower container, and hₑ is a thickness of the elastic body.

8. The multi-well plate for culturing a single microorganism of claim 1, wherein the through hole has a diameter of 1.0 to 3.0 mm.

9. The multi-well plate for culturing a single microorganism of claim 1, wherein the lid includes a gas permeable breathable membrane.

10. The multi-well plate for culturing a single microorganism of claim 1, wherein a cross-sectional shape of the through hole is a quadrangular, hexagonal, or circular shape.

11. A method for culturing a single microorganism, the method comprising:
injecting a culture medium including a single microorganism into a lower container including a bottom, a plurality of side walls, and first connection parts placed on the side walls;
connecting a perforated plate including a plurality of through holes and a plurality of second connection parts which are formed in an edge and independently connected to the first connection parts to the lower container to seal the culture medium in each of the through holes; and
covering the top of the perforated plate with a lid and performing culturing.

12. The method for culturing a single microorganism of claim 11, wherein the lower container includes one or more partition walls which divide across the container and one or more housing grooves placed on the partition wall, and
the perforated plate includes one or more divided regions which divide the through holes across the plate and one or more protruding parts which are placed in a lower portion of the divided region and connected to the housing groove.

13. The method for culturing a single microorganism of claim 11, wherein the lower container further includes an elastic body formed on surfaces of the bottom, the side wall, and the partition wall.

14. The method for culturing a single microorganism of claim 11, wherein the number (N) of single microorganisms injected into the lower container satisfies the following Relation 2: [Relation 2] $N \geq 0.1 a \left(unit:CFU/\left(a*V\right)\right) \left(100<a<50000\right)$ wherein a is the number of wells, and V is an individual capacity of the well.
